# EUROPEAN PATENT APPLICATION

(11) **EP 4 553 062 A1**
(43) Date of publication of application: **14.05.2025**
(21) Application number: 23835297.5
(22) Date of filing: 21.06.2023
(51) Int. Cl.: C07C 49/255, C08F 214/18, C08K 5/24, C08L 27/12

(54) **FLUORINE-CONTAINING COMPOUND, FLUORINE-CONTAINING COPOLYMER AND FLUORINE-CONTAINING COPOLYMER COMPOSITION**

(30) Priority: 06.07.2022 JP 2022109011
(71) Applicant: AGC INC., Chiyoda-ku, Tokyo 1008405 (JP)
(72) Inventor: USUDA, Tsukasa, Tokyo 100-8405 (JP); WATANUKI, Shun, Tokyo 100-8405 (JP); TAJIMA, Shinya, Tokyo 100-8405 (JP)
(74) Representative: Müller-Boré & Partner Patentanwälte PartG mbB
(86) International application number: PCT/JP2023/022853
(87) International publication number: WO 2024/009763

(57) **Abstract**

Provided are a fluorine-containing compound suitable to produce rubber having both excellent short-term and long-term heat resistance, a fluorine-containing copolymer using such a compound, and a fluorine-containing copolymer composition. A fluorine-containing compound represented by formula (1) below, a fluorine-containing copolymer using such a compound, and a fluorine-containing copolymer composition. In formula (1), R¹ to R³ are each independently a hydrogen atom or a fluorine atom; R⁴ is a hydrogen atom, a fluorine atom, or a C₁₋₈ monovalent organic group free from a carbonyl group; Z is an oxygen atom, a sulfur atom, or -N(R⁵)- where R⁵ is a hydrogen atom or a C₁₋₄ monovalent organic group; X is a single bond or a difluoromethylene group; Y¹ to Y⁴ are each independently a fluorine atom or a trifluoromethyl group; and Q is a C₁₋₈ perfluoroalkylene group optionally containing an etheric oxygen atom.

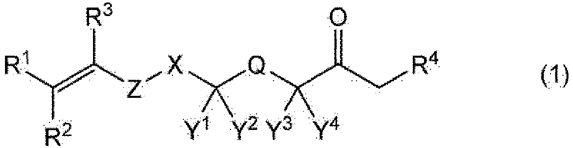

## Description

### Technical Field

The present invention relates to a fluorine-containing compound useful as a monomer constituting a fluorine-containing copolymer, a fluorine-containing copolymer using such a compound, and a composition comprising such a fluorine-containing copolymer.

### Background Art

FFKM is a fluorine-containing copolymer comprising tetrafluoroethylene (TFE) and perfluoro (alkyl vinyl ether) (e.g., perfluoro (methyl vinyl ether) (PMVE)) as monomer constituent units, and is also referred to as a perfluoroelastomer. The rubber obtained by crosslinking FFKM has excellent heat resistance, chemical resistance and the like, and is thus used in a wide range of applications (e.g., in semiconductor manufacturing, vehicles, aircrafts, general machinery, construction, chemical plants) as sealing and cushioning materials (e.g., O-rings, packings, oil seals, gaskets).

For example, a method has been known, including using, as a crosslinker, a peroxide (e.g., 2,5-dimethyl-2,5-bis(tert-butylperoxy)hexane) for crosslinking at the site of a terminal iodine atom of a monomer as a form of crosslinking. Further, from the viewpoint of increasing the rate of a crosslinking reaction, use of a bismaleimide compound as a crosslinking aid has been proposed (see PTL1).

Also known is a method including: introducing, as a monomer constituent unit, a fluorine-containing compound having a nitrile group into FFKM; and using tetraphenyl tin as a catalyst to form a cross-linked structure comprising a triazine ring by trimerization of the nitrile group (see, for example, PTL2).

Furthermore, from the viewpoint of, for instance, reducing the compression set of a cross-linked product at high temperatures of around 300°C, a method including: introducing, as a monomer constituent unit, a fluorine-containing compound having a nitrile group into FFKM; and using, as a crosslinker, a bisaminophenol compound (e.g., 2,2-bis(3-amino-4-hydroxyphenyl)hexafluoropropane) to form a cross-linked structure comprising an oxazole ring (see, for example, PTL3 and PTL4) has been proposed.

### Citation List

### Patent Literature

PTL1: WO 2021/166664
PTL2: JP S63-5409 B
PTL3: JP H02-59177 B
PTL4: WO 2021/210503

### Summary of Invention

### Technical Problem

With the expansion of rubber applications in various industries, rubbers are required to exert excellent rubber properties at higher temperatures. Further improvement of heat resistance is also desired in rubbers obtained by crosslinking FFKM.

However, even the rubbers described in PTLs 1-4 were not necessarily satisfactory enough in terms of both short-term and long-term heat resistance.

In view of such a situation, the purpose of the present invention is to provide a fluorine-containing compound suitable to produce excellent rubber having both excellent short-term and long-term heat resistance, a fluorine-containing copolymer using such a compound, and a fluorine-containing copolymer composition.

### Solution to Problem

The present invention is based on the finding that when a given fluorine-containing compound is introduced into a monomer constituent unit of FFKM, a cross-linked product obtained from a fluorine-containing copolymer using the fluorine-containing compound has both excellent short-term and long-term heat resistance.

The present invention provides the following means.
[1] A fluorine-containing compound represented by the following formula (1): 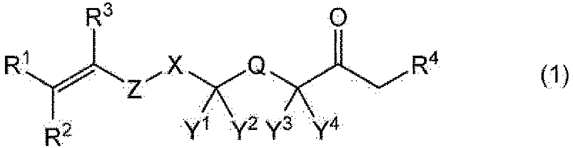 wherein R¹ to R³ are each independently a hydrogen atom or a fluorine atom; R⁴ is a hydrogen atom, a fluorine atom, or a C₁₋₈ monovalent organic group free from a carbonyl group; Z is an oxygen atom, a sulfur atom, or -N(R⁵)- where R⁵ is a hydrogen atom or a C₁₋₄ monovalent organic group; X is a single bond or a difluoromethylene group; Y¹ to Y⁴ are each independently a fluorine atom or a trifluoromethyl group; and Q is a C₁₋₈ perfluoroalkylene group optionally containing an etheric oxygen atom.
[2] The fluorine-containing compound according to [1], wherein Z is an oxygen atom.
[3] The fluorine-containing compound according to [1] or [2], wherein R¹ to R³ are each a fluorine atom and R⁴ is a hydrogen atom.
[4] A fluorine-containing copolymer comprising: a constituent unit (A) derived from tetrafluoroethylene; a constituent unit (B) derived from perfluoro (alkyl vinyl ether); and a constituent unit (C) derived from the fluorine-containing compound according to any one of [1] to [3].
[5] The fluorine-containing copolymer according to [4], wherein the constituent unit (A) is from 29.9 to 80 mass%, the constituent unit (B) is from 19.9 to 70 mass%, and the constituent unit (C) is from 0.1 to 20 mass% based on total 100 mass% of the constituent units (A) to (C).
[6] A fluorine-containing copolymer composition comprising the fluorine-containing copolymer according to [4] or [5] and a compound (D) having two or more structures (d) represented by the following formula (2): 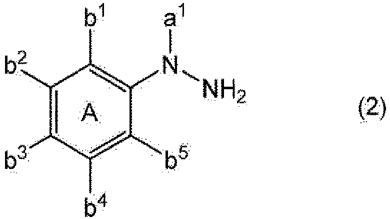 wherein -a¹ represents a single bond and is attached to a hydrogen atom, a methyl group, or an optionally substituted aromatic ring; and
   -b¹ to -b⁵ each represent a single bond, and at least one of -b¹ and -b⁵ is bonded to a hydrogen atom,
   provided that the benzene ring in formula (2) is denoted as a benzene ring A.
[7] The fluorine-containing copolymer composition according to [6], wherein the compound (D) has one or more structures in which the benzene ring A of one of the structures (d) is linked, via a single bond, to the benzene ring A of another structure (d).
[8] The fluorine-containing copolymer composition according to [6] or [7], wherein the compound (D) has a content of from 0.1 to 10 parts by mass based on 100 parts by mass of the fluorine-containing copolymer.
[9] A cross-linked fluorine-containing copolymer product obtainable from the fluorine-containing copolymer composition according to any one of [6] to [8].

### Advantageous Effects of Invention

The present invention can provide a fluorine-containing compound suitable to produce rubber having both excellent short-term and long-term heat resistance, a fluorine-containing copolymer using such a compound, and a fluorine-containing copolymer composition.

### Description of Embodiments

Here, the terms and the definitions and meanings of notations herein are given below.

The "room temperature" means from 20 to 25°C.

The term "short-term heat resistance" means that the mechanical properties, especially storage modulus, remain almost unchanged at the time of heating from room temperature to about 300°C in a test according to the "tensile vibration-non-resonance method" specified in JIS K 7244-4:1999. Specifically, the thermo-mechanical analysis described in the Examples is used for evaluation.

The term "long-term heat resistance" means that the mechanical properties are maintained for 72 hours or longer at a high temperature of about 300°C. Specifically, the hanging method described in the Examples is used for evaluation.

The content ratio between each monomer constituent unit in a fluorine-containing copolymer is calculated from the results of ¹H-NMR and ¹⁹F-NMR as measured using a nuclear magnetic resonance (NMR) measuring device. Specifically, the content ratio can be calculated by the method described in the Examples.

### [Fluorine-containing compound]

A fluorine-containing compound according to an embodiment of the present invention (hereinafter, also referred to as a "fluorine-containing compound of this embodiment") is represented by the following formula (1).

This compound is a novel fluorine-containing compound having a vinyl or fluorovinyl group as a polymerizable unsaturated bond and a carbonyl group in the main chain. Each fluorine-containing compound having such a structure may be introduced as a monomer constituent unit into FFKM to form a cross-linked fluorine-containing copolymer product having a cross-linked structure comprising an indole ring. Such a cross-linked product excels in both short-term and long-term heat resistance. The fluorine-containing compound of this embodiment may be used to constitute a fluorine-containing copolymer suitable for producing the cross-linked product.

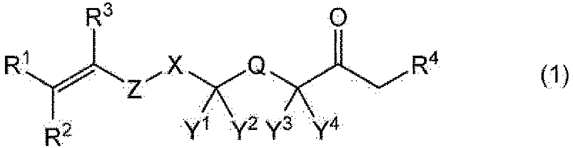

In formula (1), R¹ to R³ are each independently a hydrogen atom or a fluorine atom. From the viewpoint of polymerization reactivity, it is preferable that each is a hydrogen atom or a fluorine atom. From the viewpoint of heat resistance, each is more preferably a fluorine atom.

R⁴ is a hydrogen atom, a fluorine atom, or a C₁₋₈ monovalent organic group free from a carbonyl group. From the viewpoint of ease of synthesis of the fluorine-containing compound of this embodiment, the number of carbon atoms in the organic group is preferably from 1 to 7, more preferably from 1 to 6, and still more preferably from 1 to 5. The organic group may be linear, branched, or cyclic, and is preferably linear or branched. The organic group may comprise a heteroatom(s) such as a nitrogen, oxygen or sulfur atom(s).

R⁴ is a hydrogen atom, depending on the crosslinker used, in view of the fact that it is the cross-linking site of the fluorine-containing copolymer. In other words, the fluorine-containing compound should have an acetyl group containing R⁴ at the molecular end.

Z is an oxygen atom, a sulfur atom or -N(R⁵)- where R⁵ is a hydrogen atom or a C₁₋₄ monovalent organic group. The number of carbon atoms in the organic group is preferably from 1 to 3, more preferably 1 or 2, and still more preferably 1 from the viewpoint of ease of synthesis of the fluorine-containing compound of this embodiment. The organic group may be either linear, branched, or cyclic, and is preferably linear or branched. The organic group may comprise a heteroatom(s) such as a nitrogen, oxygen or sulfur atom(s).

Z is preferably an oxygen atom or a sulfur atom and more preferably an oxygen atom from the viewpoint of favorable rubber properties of the cross-linked fluorine-containing copolymer product in which the fluorine-containing compound of this embodiment is a monomer constituent unit.

X is a single bond or a difluoromethylene group and preferably a single bond from the viewpoint of ease of synthesis of the fluorine-containing compound of this embodiment.

Y¹ to Y⁴ are each independently a fluorine atom or a trifluoromethyl group. From the viewpoint of polymerization reactivity and heat resistance, Y¹ and Y³ are each a fluorine atom, and from the viewpoint of ease of synthesis of the fluorine-containing compound of this embodiment, each of Y¹ to Y⁴ is preferably a fluorine atom.

Q is a C₁₋₈ perfluoroalkylene group optionally containing an etheric oxygen atom. The number of carbon atoms in the perfluoroalkylene group is preferably from 1 to 7 and more preferably from 1 to 6 from the viewpoint of ease of synthesis of the fluorine-containing compound. When an etheric oxygen atoms is included, the number of oxygen atoms is preferably from 1 to 3, more preferably from 1 or 2, and still more preferably 1. Q may be linear or branched, and is preferably linear.

From the viewpoint of ease of synthesis of the fluorine-containing compound, Q is preferably a perfluoroalkylene group and particularly preferably a difluoromethylene group, i.e., in which the number of carbon atoms is 1.

Specific examples of the compound represented by formula (1) include CF₂=CFO(CF₂)₂(C=O)CH₃, CF₂=CFO(CF₂)₃(C=O)CH₃ (hereinafter, also referred to as CSM-1), CF₂=CFO(CF₂)₄(C=O)CH₃, CF₂=CFO(CF₂)₃O(CF₂)₂(C=O)CH₃, CF₂=CFOCF₂C(CF₃)FO(CF₂)₂(C=O)CH₃, or CF₂=CFOCF₂C(CF₃)FO(CF₂)₃(C=O)CH₃. Among them, CSM-1 is preferable from the viewpoint of, for instance, ease of synthesis.

### [Method for producing fluorine-containing compound]

The fluorine-containing compound of this embodiment may be obtained, for example, by reacting perfluorovinyloxy polyether carboxylic acid or its derivative with a reagent such as a Grignard reagent or organolithium reagent to form a ketone. Examples of the carboxylic acid derivative include an ester or a carboxylic acid halide. For example, a Grignard reagent (organomagnesium halide: general formula RMgX) may be used. In this case, the synthesis scheme may be applied, including substituting, with an organic group R of Grignard reagent, a substituent (terminal group) attached to the carbonyl group of perfluorovinyloxy polyether carboxylic acid or its derivative. Specifically, the compound can be synthesized by the method described in the Examples or is also available commercially.

### [Fluorine-containing copolymer]

A fluorine-containing copolymer according to an embodiment of the present invention (hereinafter, also referred to as a "fluorine-containing copolymer of this embodiment") comprises: a constituent unit (A) derived from tetrafluoroethylene (TFE); a constituent unit (B) derived from perfluoro (alkyl vinyl ether) (PAVE); and a constituent unit (C) derived from a fluorine-containing compound of this embodiment described above.

When the constituent unit (C) derived from the above-mentioned fluorine-containing compound is introduced into FFKM, a cross-linked product having both excellent short-term and long-term heat resistance can be obtained from such a fluorine-containing copolymer of this embodiment.

The monomer used as the constituent unit (A) is TFE.

The monomer used as the constituent unit (B) is PAVE.

From the viewpoint of favorable polymerization reactivity and rubber properties, the number of carbon atoms in the perfluoroalkyl group of PAVE is preferably from 1 to 10, more preferably from 1 to 8, still more preferably from 1 to 6, still more preferably from 1 to 5, and particularly preferably from 1 to 3. The perfluoroalkyl group may be linear or branched.

Specific examples of PAVE include perfluoro (methyl vinyl ether) (PMVE), perfluoro (ethyl vinyl ether) (PEVE), or perfluoro (n-propyl vinyl ether) (PPVE). One kind of PAVE may be used singly, or two or more kinds thereof may be used in combination. Among these, PMVE or PEVE is preferable and PMVE is more preferable from the viewpoint of polymerization reactivity.

The monomer of the constituent unit (C) is a fluorine-containing compound in this embodiment. One kind of the fluorine-containing compound in this embodiment may be used singly, or two or more kinds thereof may be used in combination. Among them, CSM-1 is preferable from the viewpoint of polymerization reactivity.

From the viewpoint of favorable heat resistance and rubber properties of a cross-linked product, in the fluorine-containing copolymer of this embodiment, it is preferred that the constituent unit (A) is from 29.9 to 80 mass%, the constituent unit (B) is from 19.9 to 70 mass%, and the constituent unit (C) is from 0.1 to 20 mass% based on total 100 mass% of the constituent units (A) to (C).

The content of the constituent unit (A) is preferably from 29.9 to 80 mass%, more preferably from 38.8 to 75 mass%, and still more preferably from 45.5 to 70% based on total 100 mass% of the constituent units (A) to (C).

The content of the constituent unit (B) is preferably from 19.9 to 70 mass%, more preferably from 24.8 to 60 mass%, and still more preferably from 29.5 to 50 mass% based on total 100 mass% of the constituent units (A) to (C).

The content of the constituent unit (C) is preferably from 0.1 to 20 mass%, more preferably from 0.2 to 15 mass%, and still more preferably from 0.5 to 10 mass% based on total 100 mass% of the constituent units (A) to (C).

The fluorine-containing copolymer of this embodiment may comprise another monomer-based constituent unit(s) in addition to the constituent units (A) to (C). From the viewpoint of heat resistance of the cross-linked fluorine-containing copolymer product of this embodiment, the total of the other monomer constituent units is preferably from 0 to 20 mass%, more preferably from 0 to 15 mass%, and still more preferably from 0 to 10 mass% based on total 100 mass% of all the monomer constituent units of the fluorine-containing copolymer of this embodiment. It is most preferred that no other monomers are included.

Examples of the other monomer to be a constituent unit other than the constituent units (A) to (C) include vinylidene fluoride, chlorotrifluoroethylene, hexafluoropropylene, a fluorine-containing monomer having two polymerizable unsaturated groups (hereinafter, referred to as "DVM"), 2,3,3,3-tetrafluoro-1-propene, or a hydrocarbon monomer. One kind of the other monomer may be used singly, or two or more kinds thereof may be used in combination.

For DVM, the compound represented by formula (3) below is preferred from the viewpoint of heat resistance of the cross-linked fluorine-containing copolymer product of this embodiment. DVM having two polymerizable unsaturated groups may be used as a monomer to give a branched fluorine-containing copolymer.

(CR²¹R²²=CR²³)₂R²⁴ (3)

In formula (3), R²¹, R²², and R²³ are each independently a hydrogen atom, a fluorine atom, a methyl group, or a trifluoromethyl group. A plurality of R²¹, R²², and R²³ may be the same or different from each other, but are preferably identical. From the viewpoint of polymerization reactivity, R²¹, R²², and R²³ are each preferably a hydrogen atom or a fluorine atom, all of them are more preferably hydrogen atoms or fluorine atoms, and, from the viewpoint of heat resistance, all of them are still more preferably fluorine atoms.

R²⁴ is a C₁₋₁₀ perfluoroalkylene group optionally containing an etheric oxygen atom. The number of carbon atoms in the perfluoroalkylene group is preferably from 1 to 8, more preferably from 1 to 6, and still more preferably from 1 to 4. When an etheric oxygen atoms is included, the number of oxygen atoms is preferably from 1 to 3, more preferably from 1 or 2, and still more preferably 1. R²⁴ may be linear, branched, or cyclic, and is preferably linear or branched and more preferably linear.

Specific examples of DVM include CF₂=CFO(CF₂)₂OCF=CF₂, CF₂=CFO(CF₂)₃OCF=CF₂, CF₂=CFO(CF₂)₄OCF=CF₂, CF₂=CFO(CF₂)₆OCF=CF₂, CF₂=CFO(CF₂)₈OCF=CF₂, CF₂=CFO(CF₂)₂OCF(CF₃)CF₂OCF=CF₂, CF₂=CFO(CF₂)₂O(CF(CF₃)CF₂O)₂CF=CF₂, CF₂=CFOCF₂O(CF₂CF₂O)₂CF=CF₂, CF₂=CFO(CF₂O)₃(CF(CF₃)CF₂O)₂CF=CF₂, CF₂=CFOCF₂CF(CF₃)O(CF₂)₂OCF(CF₃)CF₂OCF=CF₂, CF₂=CFOCF₂CF₂O(CF₂O)₂CF₂CF₂OCF=CF₂, CF₂=CFO(CF₂)₂CF=CF₂, CH₂=CH(CF₂)₂CH=CH₂, CH₂=CH(CF₂)₄CH=CH₂, or CH₂=CH(CF₂)₆CH=CH₂. Among them, preferred is CF₂=CFO(CF₂)₃OCF=CF₂, CF₂=CFO(CF₂)₄OCF=CF₂, or CH₂=CH(CF₂)₆CH=CH₂.

Examples of the hydrocarbon monomer include an olefin (e.g., ethylene, propylene, isobutene, 1-butene).

### [Method for producing fluorine-containing copolymer]

The fluorine-containing copolymer of this embodiment may be obtained, for example, by copolymerization of monomers to be constituent units through, for instance, emulsion polymerization, solution polymerization, or suspension polymerization in the presence of a radical polymerization initiator. The production method, however, is not particularly limited.

The radical polymerization initiator known in the production of a fluorine-containing copolymer may be used, and is selected, if appropriate, according to the polymerization process.

For emulsion polymerization in an aqueous medium such as water, a water-soluble radical polymerization initiator is preferred. Examples include persulfates (e.g., ammonium persulfate, sodium persulfate, potassium persulfate), disuccinic acid peroxide, azobisisobutylamidine dihydrochloride, tert-butyl hydroperoxide, or peroxydicarbonates. Among them, persulfates are preferred, and ammonium persulfate is more preferred. In addition, a redox-type polymerization initiator made by combining persulfates or hydrogen peroxide with a reducing agent (e.g., sodium hydrogen sulfite, sodium thiosulfate) may be used or a metal or metal compound (e.g., a small amount of iron, ferrous salt, or silver sulfate) may also be used in combination with the redox-type polymerization initiator.

Examples of the radical polymerization initiator that can be used in solution polymerization using a solvent (e.g., 1H-perfluorohexane) include an organic peroxide (e.g., bis(pentafluoropropionyl)peroxide, pivaloyl tert-butyl peroxide, diisopropyl peroxydicarbonate).

The radical polymerization initiator may be added in one portion or sequentially. The amount of radical polymerization initiator used is preferably from 0.0001 to 3 parts by mass, more preferably from 0.001 to 2 parts by mass, and still more preferably from 0.01 to 1 part by mass based on total 100 parts by mass of all the monomers to be polymerized.

A chain transfer agent may be added to the reaction system so as to adjust the moldability and mechanical properties of the fluorine-containing polymer. Examples of the chain transfer agent include: a saturated hydrocarbon compound (e.g., hexane, cyclohexane); an alkyl-containing aromatic compound (e.g., xylene, ethylbenzene); an aliphatic alcohol (e.g., methanol, isopropyl alcohol); a carbonyl-containing compound (e.g., acetic acid, methyl acetate, acetone, dimethyl carbonate, dimethylacetamide, N-methylsuccinimide, tetramethyl urea); or a nitrile compound (e.g., acetonitrile). Among them, ketones are preferred from the viewpoint of chain transfer performance and heat resistance of molded products.

A pH buffer may be added to the reaction system. Examples of the pH buffer include disodium hydrogen phosphate, sodium dihydrogen phosphate, sodium hydrogen carbonate, sodium carbonate, or a hydrate thereof.

Examples of the emulsifier used in emulsion polymerization include: a hydrocarbon emulsifier (e.g., sodium lauryl sulfate, sodium dodecylbenzenesulfonate); or a fluorine-containing emulsifier (e.g., ammonium perfluorooctanoate, sodium perfluorooctanoate, ammonium perfluorohexanoate, CF₃(CF₂)₂O(CF(CF₃)CF₂O)₂CF(CF₃)COONH₄, CF₃(CF₂)₂OCF(CF₃)CF₂OCF(CF₃)COONH₄, CF₃(CF₂)₂OCF₂CF₂OCF₂COONH₄, CF₃(CF₂)₂O(CF₂CF₂O)₂CF₂COONH₄, CF₃(CF₂)₃OCF₂CF₂OCF₂COONH₄, CF₃(CF₂)₃O(CF₂CF₂O)₂CF₂COONH₄, CF₃(CF₂)₂OCF₂CF₂OCF₂COONa, CF₃(CF₂)₂O(CF₂CF₂O)₂CF₂COONa, CF₃(CF₂)₃OCF₂CF₂OCF₂COONa, CF₃(CF₂)₃O(CF₂CF₂O)₂CF₂COONa, CF₃CF₂OCF₂CF₂OCF₂COONH₄, CF₃CF₂O(CF₂CF₂O)₂CF₂COONH₄, CF₃CF₂OCF₂CF₂OCF₂COONa, CF₃CF₂O(CF₂CF₂O)₂CF₂COONa, CF₃0CF₂CF₂CF₂OCF₂COONH₄, CF₃OCF₂CF₂CF₂OCF(CF₃)COONH₄, CF₃OCF₂CF₂CF₂OCF₂COONa, CF₃OCF₂CF₂CF₂OCF(CF₃)COONa, CF₃O(CF₂O)₃CF₂COONH₄, CF₃O(CF₂O)₃CF₂COONa, CF₃OCF(CF₃)CF₂OCF(CF₃)COONH₄, CF₃OCF(CF₃)CF₂OCF(CF₃)COONa, CF₃O(CF₂CF₂O)₂CF₂COONH₄, CF₃O(CF₂CF₂O)₂CF₂COONa). One kind of the emulsifier may be used singly, or two or more kinds thereof may be used in combination. Among them, preferred is ammonium perfluorooctanoate, CF₃(CF₂)₃OCF₂CF₂OCF₂COONH₄, CF₃(CF₂)₂OCF₂CF₂OCF₂COONH₄, CF₃CF₂OCF₂CF₂OCF₂COONH₄, or CF₃OCF₂CF₂CF₂OCF₂COONH₄.

The emulsifier may be added in one portion or sequentially. The amount of emulsifier used is preferably from 0.01 to 20 parts by mass, more preferably from 0.05 to 15 parts by mass, and still more preferably from 0.1 to 10 parts by mass based on 100 parts by mass of the aqueous medium.

Conditions such as the pressure and temperature of the polymerization reaction are set, if appropriate, according to the monomer composition and the decomposition temperature of the radical polymerization initiator. Normally, the pressure is preferably from 0.1 to 20 MPaG, more preferably from 0.3 to 10 MPaG, and still more preferably from 0.3 to 5 MPaG. The temperature is preferably from 0 to 100°C, more preferably from 10 to 90°C, and still more preferably from 20 to 85°C.

In emulsion polymerization, the fluorine-containing copolymer is obtained as latex, which can be purified by adding, if appropriate, a metal salt or inorganic acid (e.g., hydrochloric acid, sulfuric acid), and condensing by a known process such as mechanical shearing or freeze-thawing.

In the case of solution polymerization, for example, the purification may be implemented by washing with an aqueous medium such as methanol.

### [Fluorine-containing copolymer composition]

A fluorine-containing copolymer composition according to an embodiment of the present invention (hereinafter, also referred to as a "composition of this embodiment") comprises: the fluorine-containing copolymer of this embodiment described above; and a compound (D) having two or more structures (d) represented by formula (2) below.

The compound (D) serves as a crosslinker for the fluorine-containing copolymer of this embodiment, and the composition of this embodiment reacts to produce a cross-linked fluorine-containing copolymer product. Use of the compound (D) as a crosslinker makes it possible for the fluorine-containing copolymer of this embodiment to form a cross-linked structure comprising an indole ring. 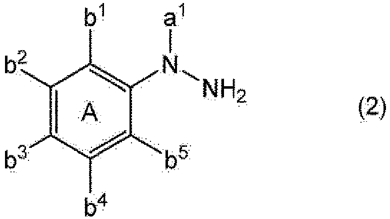 wherein -a¹ represents a single bond and is attached to a hydrogen atom, a methyl group, or an optionally substituted aromatic ring. From the viewpoint of heat resistance of cross-linked products, the aromatic ring includes a benzene ring or a naphthalene ring and is preferably a C₆₋₁₂ monocyclic or fused ring optionally containing a heteroatom. The substituent of the aromatic ring is preferably a C₁₋₆ alkyl group, more preferably a C₁₋₅ alkyl group, and still more preferably a C₁₋₄ alkyl group. The alkyl group may be linear or branched.

-b¹ to -b⁵ each represent a single bond, and at least one of -b¹ and -b⁵ is bonded to a hydrogen atom in order to form an indole ring in the crosslinking reaction. -b¹ or -b⁵ may be bonded to an aryl group attached to -a¹ to form a fused ring containing a nitrogen atom.

The compound (D) should have one or more structures in which benzene ring A of one structure (d) is linked, via a single bond, to benzene ring A of another structure (d) from the viewpoint of ease of synthesis and heat resistance of the cross-linked fluorine-containing copolymer product in this embodiment.

In addition, from the viewpoint of crosslinking reactivity, the structure (d) has a hydrazinobenzene skeleton with -a¹ bonded to a hydrogen atom, and the compound (D) preferably has two structures (d) having the hydrazinobenzene skeleton and bonded to any of -b¹ to -b⁵.

Further, it is more preferred that the compound (D) is a compound in which the benzene rings A of two hydrazinobenzene are bonded, via a single bond, to each other. The bond position of the single bond connecting the benzene rings A may be any of -b¹ to -b⁵. In such a structure, the cross-linked structure formed is very thermally stable, thereby producing a cross-linked product with better heat resistance.

Among them, from the viewpoint of thermal stability of the cross-linked structure formed and availability of raw materials, preferred is a compound having a hydrazino group at the para (p-) position relative to the single bond connecting benzene rings A. Examples include compounds (CLA-1 to CLA-3) represented by the following formulas, and CLA-1 is particularly preferred.

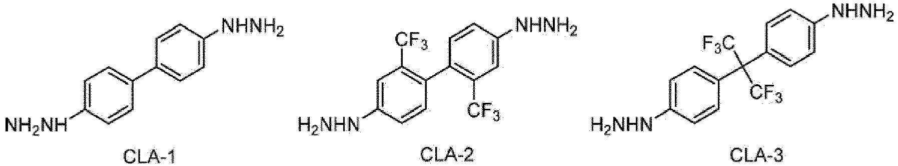

CLA-1 may be obtained as a hydrochloride produced, for example, by using 4,4'-diiodobiphenyl as a raw material and replacing the iodine atom with a hydrazino group by a known procedure. CLA-1 may also be available commercially. In addition, CLA-2 may be synthesized, for example, by the method described in the Examples, and CLA-3 can also be synthesized by the same method.

Examples of the preferred compound (D) include those in which two structures (d) are linked via one or more selected from the group consisting of an oxygen atom, a sulfur atom, a sulfonyl group, a perfluoroalkylene group, a perfluoropolyether group, and an optionally substituted divalent aromatic ring.

Other specific examples of the compound (D) are shown below.

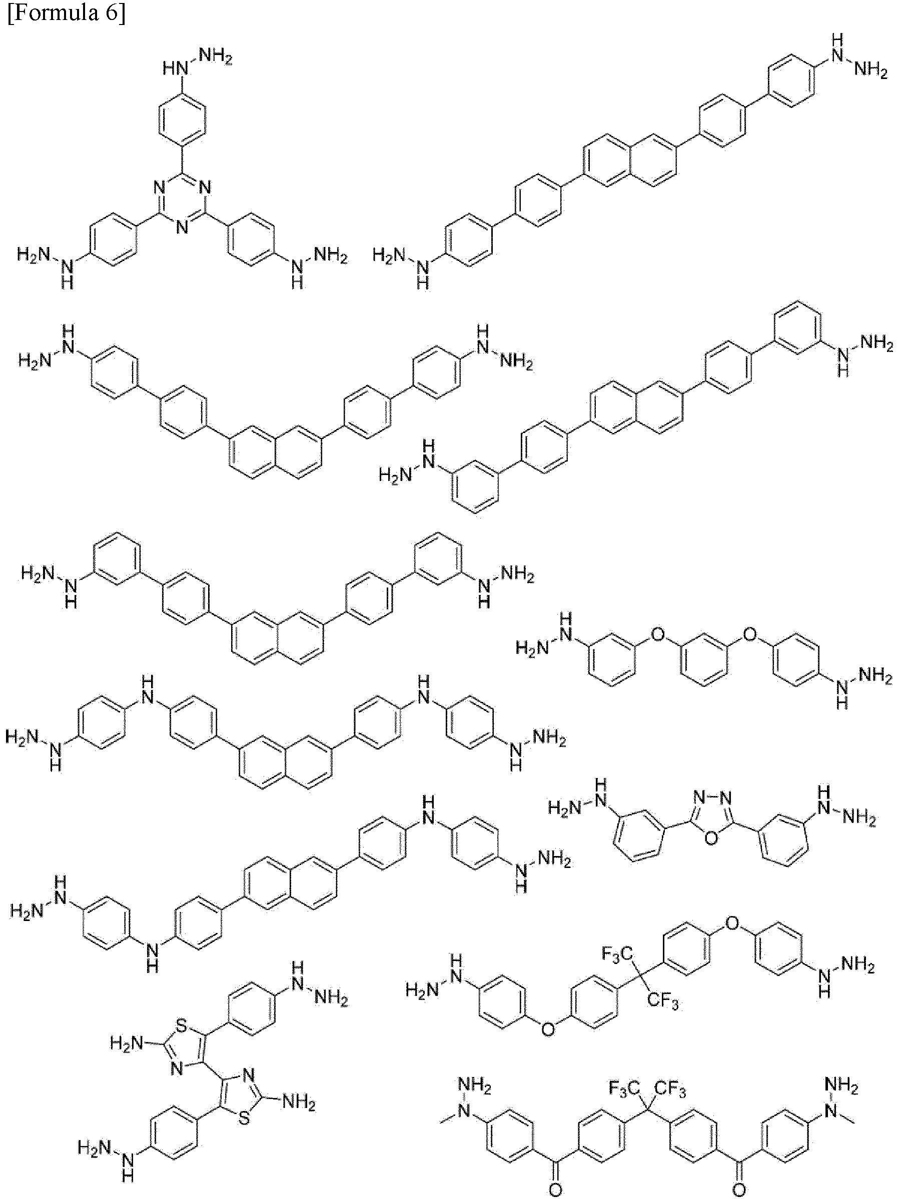

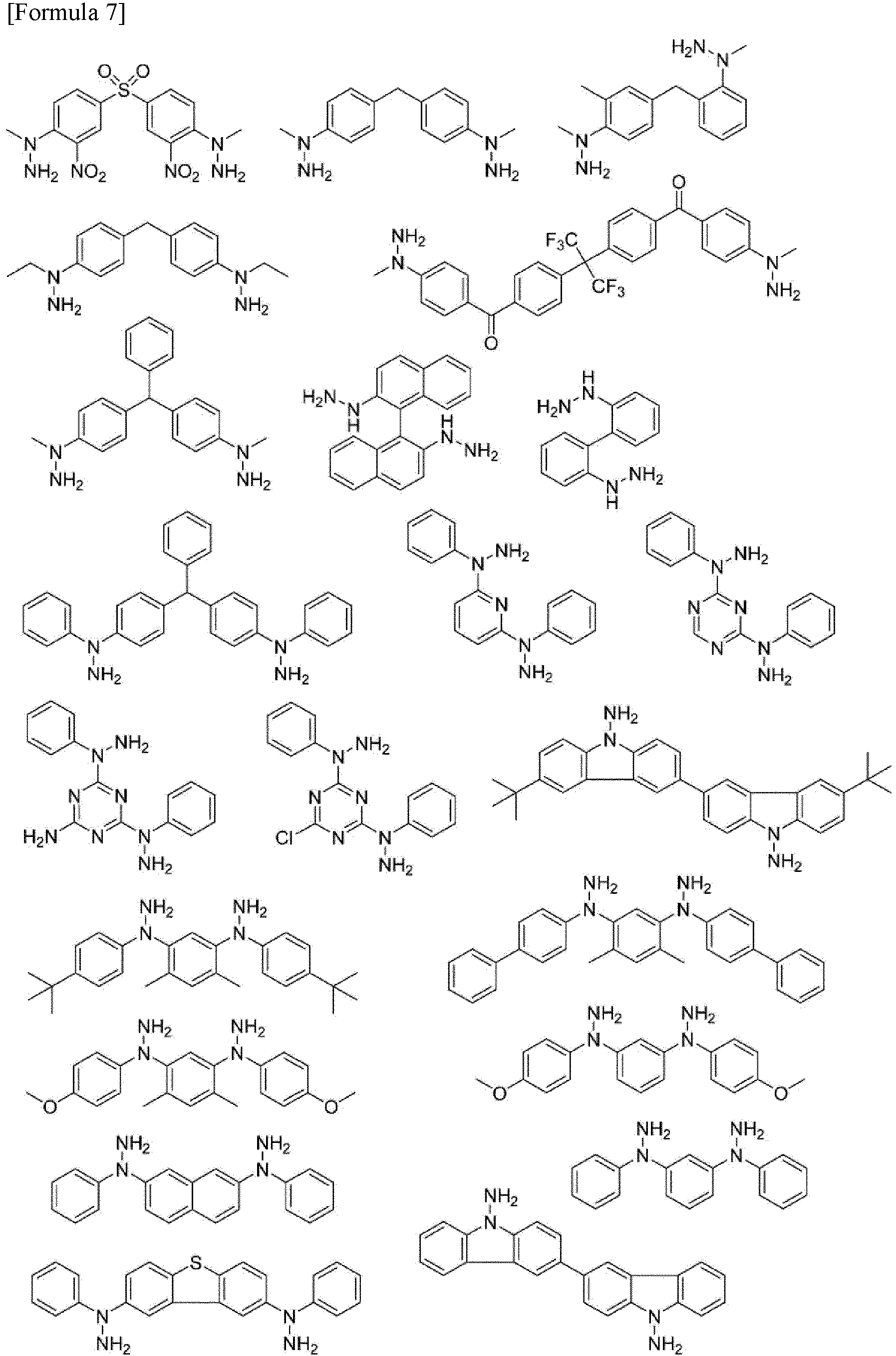

In the composition of the present embodiment, the content of compound (D) is preferably from 0.1 to 10 parts by mass, more preferably from 0.2 to 8 parts by mass, and still more preferably from 0.3 to 5 parts by mass based on 100 parts by mass of the fluorine-containing copolymer of this embodiment from the viewpoint of sufficient heat resistance and rubber properties of the cross-linked fluorine-containing copolymer product. One kind of the compound (D) may be used singly, or two or more kinds thereof may be used in combination.

The composition of this embodiment may comprise other additives in addition to the fluorine-containing copolymer of this embodiment and the compound (D) to the extent that the effect of the present invention is not impaired. Examples of the other additives include crosslinking aids, fillers, reinforcing materials, scorch retardants, mold releasing agents, acid acceptors, crown ethers, and dyes. One kind of each additive selected from those may be used singly or two or more kinds thereof may be used in combination.

A preferable crosslinking aid is an acid catalyst from the viewpoint of promoting the compound (D)-mediated crosslinking reaction of the fluorine-containing copolymer. It is considered that the inclusion of an acid catalyst in the composition of this embodiment promotes the mutual isomerization from arylhydrazone to enhydrazine as formed in the system, and also accelerates the formation of an indole ring. Examples of the acid catalyst include protonic acid or Lewis acid. Specific examples include: an aliphatic carboxylic acid (e.g., acetic acid, trifluoroacetic acid, lauric acid, stearic acid) (including a fluorine-containing fatty acid); an aromatic carboxylic acid (e.g., benzoic acid); or sulfuric acid, zinc chloride, a boron trifluoride-diethyl ether complex, polyphosphoric acid, p-toluenesulfonic acid, or trifluoromethanesulfonic acid. Among them, an aliphatic carboxylic acid, an aromatic carboxylic acid, polyphosphoric acid, or p-toluenesulfonic acid is preferred from the viewpoint of crosslinking reactivity.

The crosslinking aid may be added as an additive to the composition, or may be prepared beforehand as a quaternary ammonium chloride with the compound (D) by a known technique. Examples of a counter anion for the quaternary ammonium cation include a carboxylic acid anion or a sulfonic acid anion. From the viewpoint of crosslinking reactivity, preferred is a conjugated anion of acid with an acid dissociation constant of 4 or less in water.

Examples of the filler and reinforcing material include: a carbon material (e.g., carbon black, fullerene, carbon nanotube, graphite fluoride, carbon fluoride); a fluoropolymer (e.g., polytetrafluoroethylene, a tetrafluoroethylene-fluoroalkyl vinyl ether copolymer, a tetrafluoroethylene-hexafluoropropylene copolymer, an ethylene-tetrafluoroethylene copolymer); barium sulfate; calcium metasilicate; a metal oxide (e.g., silicon dioxide, titanium oxide, yttrium oxide, aluminum oxide); a metal carbide (e.g., silicon carbide, aluminum carbide); a metal nitride (e.g., silicon nitride, aluminum nitride); an imide filler (e.g., polyimide, polyamideimide, polyetherimide); a polyarylether ketone (e.g., polyetheretherketone (PEEK), polyetherketone (PEK), polyetherketone ketone (PEKK)); polyester; polyethersulfone; polyphenylene sulfide; clay; or talc.

Examples of the scorch retardant include a phenolic hydroxyl group-containing compounds (e.g., bisphenol A), quinones (e.g., hydroquinone), or α-methylstyrene dimers (e.g., 2,4-diphenyl-4-methyl-1-pentene).

Examples of the mold releasing agent include sodium stearate. Examples of the acid acceptor include: an aliphatic polyester; an aliphatic metal salt; or a divalent metal oxide (e.g., calcium oxide, zinc oxide, lead oxide). Examples of the crown ether include 18-crown-6.

Examples of the dye include: an inorganic pigment (e.g., zinc oxide, titanium dioxide, carbon black); or an organic pigment having a diketopyrrolopyrrole skeleton, isoindolinone skeleton, quinacridone skeleton, anthraquinone skeleton, polyarylene skeleton, or imide skeleton. Among them, an organic pigment is preferred from the viewpoint of improving heat resistance and plasma resistance as caused by their ability to quench radicals or redox active species.

The composition in this embodiment may contain another additive(s). **In** this case, the total content of the other additives is preferably more than 0 part by mass and 30 parts by mass or less, more preferably 25 parts by mass or less, and still more preferably 20 parts by mass or less based on 100 parts by mass of the fluorine-containing copolymer of this embodiment.

### [Cross-linked product]

**In** the case of crosslinking by heating, for example, the fluorine-containing copolymer may be cross-linked by heating at 80 to 300°C for 0.1 to 24 hours. Once heated, the fluorine-containing copolymer may be further heated at a different temperature. That is, after primary crosslinking, secondary crosslinking may be performed. From the viewpoint of moderate progression of the crosslinking reaction, for example, primary crosslinking at 80-200°C for 0.1-6 hours, followed by secondary crosslinking at 200-300°C for 1-24 hours, is preferred. During primary crosslinking, the mixture may be molded as needed.

Examples of the molding process include compression molding, injection molding, extrusion molding, calendering, or molding by dipping or coating, after dissolution in a solvent, into or onto, for instance, a substrate.

As described above, the cross-linked product obtained by the reaction with the composition of this embodiment comprises an indole ring-containing cross-linked structure.

Each cross-linked product comprising a ring structure in the cross-linked structure tends to have better heat resistance than a ring structure-free cross-linked products. In addition, when the ring structure contained in the cross-linked structure is an indole ring, the heat resistance is even better than when the ring structure is a benzene ring, an oxazole ring, or a triazine ring. In particular, the former excels in both short-term and long-term heat resistance.

The cross-linked fluorine-containing copolymer product of this embodiment is suitable as sealings and cushioning materials such as O-rings, V-rings, packings, oil seals, gaskets, diaphragms, and sheets. The cross-linked product is also preferably applicable in various applications such as heat-resistant and chemical-resistant sealing materials, heat-resistant and oil-resistant sealing materials, electric wire coating materials, sealing materials for semiconductor devices, corrosion resistant rubber coatings, sealing materials for urea resistant grease, rubber coatings, adhesive rubbers, hoses, tubes, calender sheets (rolls), sponges, rubber rolls, oil drilling members, heat-dissipating sheets, solution cross-linked components, rubber sponges, bearing seals, linings, insulating sheets for automobiles, insulating sheets for electronic equipment, rubber bands for watches, packing for endoscopes, bellows hoses, packing/valves for water heaters, fenders, fibers/non-woven fabrics (e.g., protective clothing), base sealing materials, rubber gloves, stators of uniaxial eccentric screw pumps, parts for urea-SCR systems, anti-vibration agents, damping agents, sealants, additives for other materials, and toys.

### Examples

Hereinafter, the present invention will be described more specifically with reference to Examples. The present invention, however, is not limited to these Examples below. Various modifications are allowed without departing from the subject matter of the present invention.

### [Compounds used]

Abbreviations of the various compounds used are explained below.
·PX1: TFE/PMVE copolymer with an iodine atom at an end; molar ratio between TFE and PMVE-derived constituent units: 66/34; iodine atom content: 0.15 mass% (for the production method, see Example 1 of the Examples in WO 2018/225586)
·PX2: TFE/PMVE/CF₂=CFO(CF₂)₃OCF=CF₂ copolymer with an iodine atom at an end; molar ratio between TFE, PMVE, and CF₂=CFO(CF₂)₃OCF=CF₂-derived constituent units: 65.9/34.0/0.1; iodine atom content: 0.20 mass% (for the production method, see copolymer 4 of the Examples in WO 2020/184427 (provided that agglomeration with potassium aluminum sulfate was changed to agglomeration with nitric acid))
·PX3: TFE/PMVE/CF₂=CFO(CF₂)₃OCF=CF₂ copolymer with an iodine atom at an end; molar ratio between TFE, PMVE, and CF₂=CFO(CF₂)₃OCF=CF₂-derived constituent units: 71.40/28.43/0.17; iodine atom content: 0.10 mass% (for the production method, see copolymer 1 of the Examples in WO 2020/184427)
·P25B: 2,5-dimethyl-2,5-bis(*tert*-butylperoxy)hexane; "PERHEXA (registered trademark) 25B", manufactured by NOF Corporation
·BOAP: 2,2-bis(3-amino-4-hydroxyphenyl)hexafluoropropane, manufactured by TOKYO CHEMICAL INDUSTRY CO., LTD.
·TAIC (registered trademark): triallyl isocyanurate, manufactured by TOKYO CHEMICAL INDUSTRY CO., LTD.
·C6-DV: 1,6-divinyl perfluorohexane, manufactured by Tosoh Finechem Corporation
·FB-BMI: bismaleimide compound represented by the following formula (see PTL1) 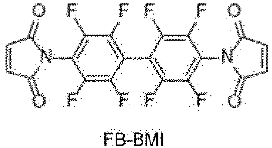
·2E4MZ: 2-ethyl-4-methylimidazole, manufactured by TOKYO CHEMICAL INDUSTRY CO., LTD.

### [Analysis and measurement protocol]

### <Nuclear magnetic resonance (NMR) measurement>

The synthesized compounds were structurally analyzed by measuring ¹H-NMR and ¹⁹F-NMR while using a nuclear magnetic resonance spectrometer ("JNM-AL300", manufactured by JEOL Ltd.; hereinafter, the same).

### <Mass spectrometry (MS)>

In addition, each molecular weight was determined by gas chromatography-mass spectrometry ("GCMS-QP2010 Ultra", manufactured by Shimadzu Corporation; chemical ionization (CI) method).

### <Content ratio between each monomer constituent unit in fluorine-containing copolymer>

The content ratio (mass ratio and molar ratio) between each monomer constituent unit in a fluorine-containing copolymer was calculated from the results of ¹H-NMR and ¹⁹F-NMR as measured using an NMR measuring device.

Here, 1H-perfluorohexane solution containing a fluorine-containing copolymer and 1,4-bis(trifluoromethyl)benzene (standard substance) was used as measurement samples.

The content ratio of PMVE constituent unit in the fluorine-containing copolymer was converted from the integral ratio between the peak attributable to the fluorine atom of the trifluoromethyl group of PMVE and the peak attributable to the fluorine atom of the trifluoromethyl group of 1,4-bis(trifluoromethyl)benzene.

The content ratio of the constituent unit of CSM-1 in the fluorine-containing copolymer was converted from the integral ratio between the peak attributable to the hydrogen atom of the methyl group of CSM-1 unit and the peak attributable to the hydrogen atom of the aromatic ring of 1,4-bis(trifluoromethyl)benzene.

The content ratio of 8CNVE constituent unit in the fluorine-containing copolymer was converted from the integral ratio between the peak attributable to the fluorine atom of the difluoromethylene group adjacent to the nitrile group of 8CNVE and the peak attributable to the fluorine atom of the trifluoromethyl group of 1,4-bis(trifluoromethyl)benzene.

The content ratio of TFE in the fluorine-containing copolymer was determined as the remainder of the content ratio of the PMVE constituent unit and the content ratio of CSM-1 constituent unit.

### [Synthesis of a monomer]

### (Synthesis Example 1) Synthesis of CSM-1

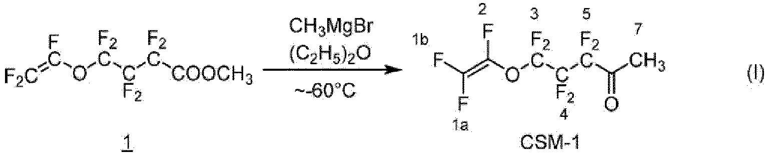

To a 2-L four-necked flask were added 107 g (350mmol) of raw material compound (1) and 750 mL of diethyl ether, and the reaction solution was cooled to -65°C or less in a dry ice/acetone bath. The reaction solution was kept at -60°C or less and 436 mL (about 3 mol/L) of diethyl ether solution containing 35 mass% methylmagnesium bromide was added dropwise over 2 hours, and the reaction continued for another 4 hours. The reaction solution was kept at -38°C or less, and a mixture of 150 mL of hydrochloric acid at a concentration of 36 mass% and 150 mL of tetrahydrofuran cooled to 5°C was added dropwise over 2.5 hours.

The product was brought to room temperature and separated by adding 170 mL of ion-exchanged water, and the organic phase was collected. The aqueous phase was admixed with 170 mL of diethyl ether to perform extraction and the resulting organic phase was combined with the previous organic phase. The organic phase obtained was washed with 170 mL of 5 mass% sodium bicarbonate aqueous solution, followed by 170 mL of saturated brine, and then admixed with anhydrous sodium sulfate and dried. After the residue was removed by filtration under reduced pressure, the product was concentrated using an evaporator at 35°C under reduced pressure (27 kPa). The concentrate was transferred into a 200-mL recovery flask with a Vigreux column loaded thereon, and purified by vacuum distillation to give 68 g (68% yield) of CSM-1.

Data in the identification of CSM-1 are as follows. Note that the sign given to the fluorine atom (F) corresponds to the sign in the above formula (I).
¹H-NMR (CDCl₃): δ [ppm] 2.46 (s, 3H)
¹⁹F-NMR (CDCl₃): δ [ppm] -85.3 (m, 2F, F-3), -113.6 (dd, 1F, J = 65.6, 82.4 Hz, F-1b), -121.4 (t, 2F, J = 9.16 Hz, F-5), -121.8 (ddt, 1F, J = 6.10, 83.9, 112.9, F-1a), -126.5 (s, 2F, F-4), -135.4 (ddt, 1F, J = 6.10, 65.6, 112.9, F-2)
MS (CI) m/z: 291 (M+H⁺)

### [Synthesis of crosslinkers]

### (Synthesis Example 2-1) Synthesis of CLA-1

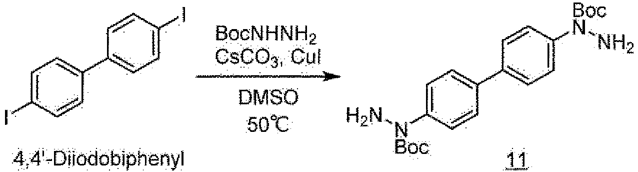

To a 1-L four-necked flask were added, under a nitrogen atmosphere, 25.0 g (1.6 mmol) of 4,4'-diiodobiphenyl, 170 mL of dimethyl sulfoxide (DMSO), 19.5 g (148 mmol) of tert-butyl carbazate (BocNHNH₂), 1.17 g (6.14 mmol) of copper (I) iodide, and 60.2 g (185 mmol) of cesium carbonate, and the reaction solution was reacted in an oil bath at a liquid temperature of 54°C for 21 hours.

The reaction solution was brought to room temperature, 600 mL of ion exchange water was added, and the mixture was stirred for 30 minutes. The precipitated solid was collected by filtration under reduced pressure and dried under reduced pressure to give a solid crude product.

The solid crude product and the filtrate were each purified by heat reflux and vacuum drying, and combined to give 12.4 g (49% yield) of compound (2) as a pale yellow solid.

Data in the identification of compound (2) are as follows.
¹H-NMR (DMSO-d₆): δ [ppm] 7.58 (d, 4H, J = 8.54 Hz, Ar-H), 7.54 (d, 4H, J = 8.54 Hz, Ar-H), 5.09 (s, 4H, NH₂), 1.47 (s, 18H, N-Boc)

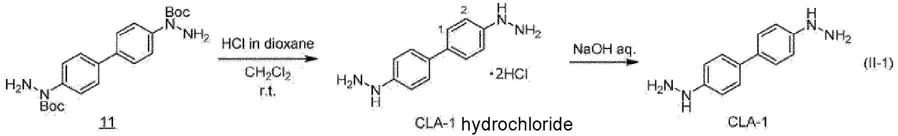

Next, 8.87 g (21.4 mmol) of compound (11) and 107 mL of dichloromethane were added to a 500-mL four-necked flask under a nitrogen atmosphere. With the mixture cooled in an ice bath, 93 mL (about 4 mol/L) of hydrogen chloride/1,4-dioxane solution was added dropwise, and the temperature was returned to room temperature and the reaction was allowed to proceed for 14 hours. The precipitated solid was collected by filtration under reduced pressure, washed with diethyl ether, and dried under reduced pressure to give 5.76 g (94% yield) of CLA-1 hydrochloride.

Under a nitrogen atmosphere, 5.00 g (17.35 mmol) of CLA-1 hydrochloride, 30 mL of dichloromethane, and 20 mL of 10 mass% sodium hydroxide aqueous solution were added to a 100-mL four-necked flask, and the mixture was stirred at room temperature for 1 hour. The precipitated solid was collected by filtration under reduced pressure, washed with ionexchange water, and dried under reduced pressure to give 3.36 g (90% yield) of CLA-1.

Data in the identification of CLA-1 hydrochloride and CLA-1 are as follows. Note that the sign given to the hydrogen atom (H) corresponds to the sign in the above formula (II-1).
<CLA-1 hydrochloride>
   ¹H-NMR (DMSO-d₆): δ [ppm] 10.3 (s, 6H, -NH₃Cl), 8.35 (s, 2H, -NH-), 7.57 (d, 4H, J = 8.54 Hz, H-1), 7.05 (d, 4H, J = 8.54 Hz, H-2)
<CLA-1>
   ¹H-NMR (DMSO-d₆): δ [ppm] 7.31 (d, 4H, J = 8.54 Hz, Ar-H), 6.79 (d, 4H, J = 8.54 Hz, Ar-H), 6.64 (s, 2H), 3.93 (s, 4H)

### (Synthesis Example 2-2) Synthesis of CLA-2 (hydrochloride)

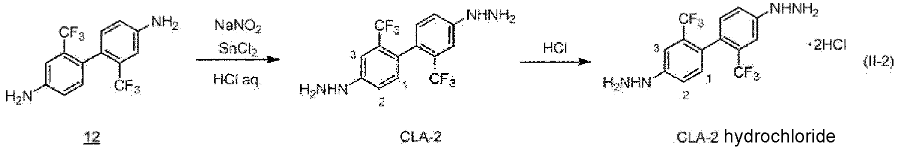

To a 100-mL four-necked flask was added 14.1 mL of hydrochloric acid at a concentration of 20 mass%, the flask was cooled in an ice bath, and 2.00 g (6.25 mmol) of compound (12) (manufactured by TOKYO CHEMICAL INDUSTRY CO., LTD.) was added with stirring. While keeping the reaction mixture at 2.5°C or less, an aqueous solution prepared by dissolving 0.89 g (12.9 mmol) of sodium nitrite in 20 mL of ion-exchanged water was added dropwise over 2 hours, and the reaction mixture was stirred for another 30 minutes. Next, a mixture of 6.85 g (30.4 mmol) of tin (II) chloride dihydrate and 8.7 mL of hydrochloric acid at a concentration of 36 mass% was added dropwise over 2 hours and stirred for another 45 minutes.

The precipitated solid was collected by filtration under reduced pressure and dried under reduced pressure at 35°C to give 0.485 g of a crude product. The crude product was admixed with 20 mL of chloroform and 11 mL of 0.5 mol/L sodium hydroxide aqueous solution, and the organic phase was separated. The aqueous phase was admixed with 20 mL of chloroform to perform re-extraction, and the resulting organic phase was combined with the previous organic phase. The organic phase obtained was admixed with anhydrous sodium sulfate and dried. After the residue was removed by filtration under reduced pressure, the organic phase was concentrated using an evaporator at 30°C under reduced pressure. The concentrate was separated and purified by silica gel column chromatography ("Silica gel 60 (spherical) NH₂", manufactured by KANTO CHEMICAL CO,INC., 16 g; mobile phase: chloroform; the same applies to the following) to give 97.6 mg of CLA-2.

To a 50-mL recovery flask were added 97.6 mg of CLA-2, 4 mL of diethyl ether, and 1 mL of approximately 1 mol/L hydrogen chloride diethyl ether solution, and the mixture was stirred.

The precipitated solid was collected by filtration under reduced pressure, and dried under reduced pressure to give 74.7 mg (3% yield) of CLA-2 hydrochloride.

Data in the identification of CLA-2 and CLA-2 hydrochloride are as follows. Note that the sign given to the hydrogen atom (H) corresponds to the sign in the above formula (II-2).
<CLA-2>
   ¹H-NMR (CDCl₃): δ [ppm] 7.18 (d, 2H, J = 2.56 Hz, H-3), 7.12 (d, 2H, J = 7.68 Hz, H-1), 6.94 (dd, 2H, J = 2.56, 8.54 Hz, H-2), 5.40 (brs, 2H, -NHNH₂), 3.66 (brs, 4H, -NHNH₂)
   ¹⁹F-NMR (CDCl₃): δ [ppm] -59.0 (s, 6F, -CF₃)
<CLA-2 hydrochloride>
   ¹H-NMR (DMSO-d₆): δ [ppm] 10.39 (brs, 6H, -NHNH₃Cl), 8.81 (brs, 2H, - NHNH₃Cl), 7.39 (d, 2H, J = 2.56 Hz, H-3), 7.3-7.2 (m, 4H, H-1 and H-2)
   ¹⁹F-NMR (DMSO-d₆): δ [ppm] -56.7 (s, 6F, -CF₃)

### (Synthesis Example 2-3) Synthesis of CLA-3 (hydrochloride)

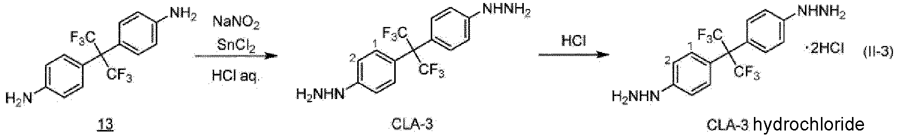

To a 100-mL four-necked flask was added 6.8 mL of hydrochloric acid at a concentration of 20 mass%, the flask was cooled in an ice bath, and 1.00 g (2.99 mmol) of compound (13) (manufactured by TOKYO CHEMICAL INDUSTRY CO., LTD.) was added with stirring. While keeping the reaction mixture at 2.0°C or less, an aqueous solution prepared by dissolving 0.43 g (6.16 mmol) of sodium nitrite in 9.5 mL of ion-exchanged water was added dropwise over 2 hours, and the reaction mixture was stirred for another 1 hour. Next, a mixture of 3.28 g (14.5 mmol) of tin (II) chloride dihydrate and 4.2 mL of hydrochloric acid at a concentration of 36 mass% was added dropwise over 2 hours and stirred for another 30 minutes.

The precipitated solid was collected by filtration under reduced pressure and dried under reduced pressure at 35°C to give 0.269 g of a crude product. The crude product was admixed with 10 mL of chloroform and 6 mL of 0.5 mol/L sodium hydroxide aqueous solution, and the organic phase was separated. The aqueous layer was admixed with 15 mL of chloroform to perform re-extraction, and the resulting organic phase was combined with the previous organic phase. The organic phase obtained was admixed with anhydrous sodium sulfate and dried. After the residue was removed by filtration under reduced pressure, the organic phase was concentrated using an evaporator at 30°C under reduced pressure. The concentrate was separated and purified by silica gel column chromatography to give 22.8 mg of CLA-3.

To a 10-mL recovery flask were added 22.8 mg of CLA-3, 1 mL of diethyl ether, and 0.25 mL of approximately 1 mol/L hydrogen chloride diethyl ether solution, and the mixture was stirred.

The precipitated solid was collected by filtration under reduced pressure, and dried under reduced pressure to give 14.9 g (1% yield) of CLA-3 hydrochloride as a pale yellow solid.
<CLA-3>
   ¹H-NMR (CDCl₃): δ [ppm] 7.2-7.3 (m, 4H, H-1), 6.78 (d, 4H, J = 9.39 Hz, H-2), 5.31 (brs, 2H, -NHNH₂), 3.60 (brs, 4H, -NHNH₂)
   ¹⁹F-NMR (CDCl₃): δ [ppm] -64.8 (s, 6F, -CF₃)
<CLA-3 hydrochloride>
   ¹H-NMR (DMSO-d₆): δ [ppm] 10.34 (brs, 6H, -NHNH₃Cl), 8.65 (brs, 2H, - NHNH₃Cl), 7.23 (d, 4H, J = 8.54 Hz, H-1), 7.03 (d, 4H, J = 8.54 Hz, H-2)
   ¹⁹F-NMR (DMSO-d₆): δ [ppm] -62.9 (s, 6F, -CF₃)

### [Production of fluorine-containing copolymers]

### (Synthesis Example 3-1) Emulsion polymerization (1)

The inside of a stainless steel autoclave having a 200-mL internal volume equipped with a stirrer was degassed. Next, 82 g of ion-exchanged water, which had been degassed under reduced pressure beforehand, 0.023 g (0.065 mmol) of disodium hydrogen phosphate dodecahydrate, 10.2 g (8.3 mmol) of 30 mass% CF₃CF₂OCF₂CF₂OCF₂COONH₄ aqueous solution, 0.062 g (0.27 mmol) of ammonium persulfate, and 0.082 g (0.27 mmol) of CSM-1 were added, and the gas phase was replaced with nitrogen. While the reaction mixture was stirred at 300 rpm, 1.4 g (14 mmol) of TFE, and 6.5 g (39 mmol) of PMVE were put into the autoclave under pressure, and the temperature of the liquid was raised to 80°C. The internal pressure of the autoclave was 0.8 MPaG. The liquid was stirred at 300 rpm for 4 hours while maintaining the temperature. The internal pressure was dropped to 0.6 MPaG. After the residual gas in the gas phase was discharged, the autoclave was opened to obtain latex containing a fluorine-containing copolymer.

The latex was frozen and then thawed to make the fluorine-containing copolymer agglomerated. The fluorine-containing copolymer was filtered, washed with ion-exchanged water, then with methanol, and vacuum-dried at 50°C to give 1.9 g of white fluorine-containing copolymer (P1).

The content ratio (mass ratio) of TFE, PMVE, and CSM-1-derived constituent units in the obtained fluorine-containing copolymer (P1) was 56.7/42.3/1.0 (molar ratio: 69.0/30.6/0.4).

### (Synthesis Example 3-2) Emulsion polymerization (2)

Latex containing a fluorine-containing copolymer was produced by polymerization in the same manner as in Synthesis Example 3-1, except that the amount of ammonium persulfate was set to 0.030 g (0.13 mmol) and the liquid temperature was maintained at 65°C after TFE and PMVE were put into the autoclave under pressure in Synthesis Example 3-1. Then, 0.68 g of white fluorine-containing copolymer (P2) was obtained. Note that during the polymerization reaction, the internal pressure of the autoclave decreased from 0.7 MPaG to 0.6 MPaG.

The content ratio (mass ratio) of TFE, PMVE, and CSM-1-derived constituent units in the obtained fluorine-containing copolymer (P2) was 52.8/40.8/6.4 (molar ratio: 68.2/29.1/2.7).

### (Synthesis Example 3-3) Solution polymerization (1)

To a stainless steel autoclave having a 200-mL internal volume equipped with a stirrer were added a solution containing 1.2 g (3.8 mmol) of CSM-1 and 0.5 g (1.5 mmol) of bis(pentafluoropropionyl)peroxide in 2.8 mass% 1H-perfluorohexane solution, and 57 g of 1H-perfluorohexane, and the mixture was freeze-degassed twice. While the reaction mixture was stirred at 300 rpm, 15 g (0.15 mol) of TFE and 103 g (0.62 mmol) of PMVE were put into the autoclave under pressure, and the temperature of the liquid was maintained at 28°C. The internal pressure of the autoclave was 0.7 MPaG. After 24 hours of stirring at 300 rpm while maintaining the liquid temperature, the internal pressure decreased to 0.5 MPaG. After the residual gas in the gas phase was discharged, the autoclave was opened to obtain a solution containing a fluorine-containing copolymer.

The resulting solution containing a fluorine-containing copolymer was vacuum-dried to give a viscous liquid. This liquid was dissolved in 200 mL of 1H-perfluorohexane, and the solution was added to 1 L of methanol and stirred for 30 minutes to produce a white solid. The solid was separated from the supernatant by using a centrifuge. The resulting solid was vacuum-dried at 50°C to give 37.1 g of white fluorine-containing copolymer (P3).

The content ratio (mass ratio) of TFE, PMVE, and CSM-1-derived constituent units in the obtained fluorine-containing copolymer (P3) was 56.1/42.8/1.1 (molar ratio: 68.5/30.5/1.0).

### (Synthesis Example 3-4) Solution polymerization (2)

A solution containing a fluorine-containing copolymer was produced by polymerization in the same manner as in Synthesis Example 3-3, except that the amount of CSM-1 was changed to 12 g (38 mmol), and the other conditions were the same as in Synthesis Example 3-3. Then, 3.2 g of white fluorine-containing copolymer (P4) was obtained. Note that during the polymerization reaction, the internal pressure of the autoclave decreased from 0.7 MPaG to 0.5 MPaG.

The content ratio (mass ratio) of TFE, PMVE, and CSM-1-derived constituent units in the obtained fluorine-containing copolymer (P4) was 52.3/38.5/9.2 (molar ratio: 69.3/21.7/9.0).

### (Synthesis Example 3-5) Modification (1) of FFKM by CSM-1

To a stainless steel autoclave having a 200-mL internal volume equipped with a stirrer were added 34 g of PX1, 32 g (0.11 mol) of CSM-1, 0.88 g (2.2 mmol) of lauroyl peroxide, and 135 g of 1H-perfluorohexane. Next, the mixture was freeze-degassed twice. The reaction solution was stirred at 300 rpm, the temperature of the solution was raised to 90°C, and the solution was stirred at 300 rpm for 4 hours while maintaining the temperature. The autoclave was cooled to room temperature and opened to obtain a solution containing a fluorine-containing copolymer.

The resulting solution containing a fluorine-containing copolymer was vacuum-dried to give a white solid. This solid was dissolved in 1 L of 1H-perfluorohexane, and the solution was added to 5 L of methanol and stirred for 5 minutes to produce a white solid. The solid was filtered, washed with methanol, and vacuum-dried at 50°C to give 29 g of white fluorine-containing copolymer (P5) (CSM-1 content ratio: 0.4 mass%).

### (Synthesis Example 3-6) Modification (2) of FFKM by CSM-1

A solution containing a fluorine-containing copolymer was produced in the same manner as in Synthesis Example 3-5, except that PX2 was used instead of PX1, and the other conditions were the same as in Synthesis Example 3-5. Then, 24 g of white fluorine-containing copolymer (P6) (CSM-1 content ratio: 0.4 mass%) was obtained.

### (Synthesis Example 3-7) Fluorine-containing copolymer with nitrile group

After the inside of a stainless steel autoclave having an internal volume of 2 L equipped with a stirrer was degassed, 880 g of 30 mass% C₂F₅O(CF₂)₂OCF₂COONH₄ aqueous solution, 7.3 g (18.8 mmol) of CF₂=CFOCF₂CF(CF₃)OCF₂CF₂CN (hereinafter, referred to as 8CNVE), 15.9 g of an aqueous solution containing 5 mass% disodium hydrogen phosphate dodecahydrate were added, and the gas phase was replaced by nitrogen. While the reaction mixture was stirred at 375 rpm, 137 g (1.37 mol) of TFE and 635 g (3.82 mmol) of PMVE were put into the autoclave under pressure, and the temperature of the liquid was raised to 80°C. The internal pressure of the autoclave was 0.90 MPaG. While the liquid temperature was maintained, 28 mL of 3 mass% ammonium persulfate aqueous solution was added, and polymerization was initiated.

When the internal pressure decreased to 0.89 MPaG, 119.3 g of TFE, 3.7 g of 8CNVE, 74 g of PMVE, and 3.7 g of 8CNVE were added under pressure in this order. After this operation was repeated 9 cycles, 119.3 g of TFE was injected under pressure and allowed to react for 375 minutes. When the polymerization rate decreased, 3 mass% ammonium persulfate aqueous solution was added as needed. The total amount of 3 mass% ammonium persulfate aqueous solution added after the start of polymerization was 35 mL.

The autoclave was cooled and the liquid temperature was lowered to 10°C to obtain latex containing a fluorine-containing copolymer.

The latex was added to 5 mass% potassium aluminum sulfate aqueous solution to agglomerate the fluorine-containing copolymer. The fluorine-containing copolymer was filtered, washed with ion-exchanged water, and vacuum-dried at 50°C to give a white fluorine-containing copolymer (P7).

The content ratio (mass ratio) of TFE, PMVE, and 8CNVE-derived constituent units in the obtained fluorine-containing copolymer (P7) was 56.8/41.3/1.9 (molar ratio: 69.1/30.3/0.6).

### [Production of cross-linked products]

### (Example 1)

100 parts by mass of the fluorine-containing copolymer (P1), 2.5 parts by mass of CLA-1, and 2.5 parts by mass of acetic acid were kneaded in a twin-screw kneader ("HAAKE (registered trademark) MiniLab3", manufactured by Thermo Fisher Scientific Inc.). The kneaded composition was heat-pressed in a heat compression press machine ("SA-401", manufactured by TESTER SANGYO CO., LTD.) using a mold of 100 mm in length, 60 mm in width, and 1 mm in thickness for 3 hours at 90°C under atmospheric conditions at a pressure of 1 MPa for primary crosslinking. The cross-linked product containing an indole ring structure was obtained by further heating the composition in an oven ("KDF-75", manufactured by YAMATO SCIENTIFIC CO., LTD.) at 240°C for 3 hours for secondary crosslinking.

### (Examples 2 to 10)

Cross-linked products were produced in the same manner as in Example 1, while using the blend composition, primary crosslinking and secondary crosslinking conditions shown in Table 1 below.

Note that in Example 10, the secondary crosslinking was performed by heating at 90°C for 2 hours, raising the temperature to 200°C over 2 hours, heating at 200°C for 4 hours, raising the temperature to 305°C over 2 hours, and heating at 305°C for 12 hours.

### [Evaluation of heat resistance of cross-linked products]

Test pieces (20-mm long, 4-mm wide, and 1-mm thick) cut out from the cross-linked fluorine-containing copolymer product obtained in each Example were evaluated for heat resistance in the following two methods. Tables 1 shows the evaluation results. Examples 1 to 6 are Working Examples, and Examples 7 to 10 are Comparative Examples.

### <Thermo-mechanical analysis (TMA)>

A thermo-mechanical analyzer ("TA7000", manufactured by Hitachi High-Tech Science Corporation) was used in accordance with JIS K 7244-4:1999, and each test piece was tested by raising the temperature from the starting temperature of -40°C to 330°C at 5°C/min. The test piece was then held at 330°C for 30 minutes, observed for the presence or absence of rupture, and measured for the storage modulus. The test results were evaluated using the following evaluation criteria.

### (Evaluation criteria)

A: The test piece was not ruptured and no change in storage modulus was observed (no decrease).
B: The test piece was not ruptured, but the storage modulus decreased.
C: The test piece was ruptured.

The heat resistance evaluation by TMA can be said to be an index of short-term heat resistance, and is judged as follows: Grade A is good, Grade B is somehow good, and Grade C is poor.

### <Hanging method>

Each test piece was hung through a stainless steel wire (diameter: 1 mm) at a site 3 mm distant from one end in the vertical direction, and was placed in an oven ("KDF-75", manufactured by YAMATO SCIENTIFIC CO., LTD.). The test piece was heated under a nitrogen atmosphere in the respective conditions shown in Table 1, and then taken out and observed for the presence or absence of deformation. The test results were evaluated using the following evaluation criteria.

### (Evaluation criteria)

A: The test piece was not deformed.
C: The test piece was deformed.

The heat resistance evaluation by the hanging method can be said to be an index of long-term heat resistance, and is judged as follows: Grade A is good and Grade C is poor.

**Table 1**

| Example | | | 1 | 2 | 3 | 4 | 5 | 6 | 7 | 8 | 9 | 10 |
|---|---|---|---|---|---|---|---|---|---|---|---|---|
| Blended component [parts by mass] | | | | | | | | | | | | |
| | Fluorine-containing copolymer | | | | | | | | | | | |
| | | P1 | 100 | 100 | 100 | - | - | - | - | - | - | - |
| | | P2 | - | - | - | 100 | 100 | 100 | - | - | - | - |
| | | PX2 | - | - | - | - | - | - | 100 | - | - | - |
| | | PX3 | - | - | - | - | - | - | - | 100 | 100 | - |
| | | P7 | - | - | - | - | - | - | - | - | - | 100 |
| | Crosslinker | | | | | | | | | | | |
| | | CLA-1 | 2.5 | 0.4 | 0.4 | 2.5 | 1.5 | 1.5 | - | - | - | - |
| | | P25B | - | - | - | - | - | - | 1 | 1 | 1 | - |
| | | BOAP | - | - | - | - | - | - | - | - | - | 0.8 |
| | Crosslinking aid | | | | | | | | | | | |
| | | Acetic acid | 2.5 | 0.4 | - | 2.5 | 1.5 | - | - | - | - | - |
| | | Lauric acid | - | - | 0.4 | - | - | 1.5 | - | - | - | - |
| | | TAIC | - | - | - | - | - | - | 2 | - | - | - |
| | | C6-DV | - | - | - | - | - | - | - | 2 | - | - |
| | | FB-BMI | - | - | - | - | - | - | - | - | 2 | - |
| | Acid scavenger | | | | | | | | | | | |
| | | 2E4MZ | - | - | - | - | - | - | 1 | 1 | 1 | - |

| Primary crosslinking | | | | | | | | | | | | |
|---|---|---|---|---|---|---|---|---|---|---|---|---|
| | Temperature [°C] | | 90 | 90 | 110 | 90 | 90 | 110 | 170 | 170 | 170 | 180 |
| | Time [h] | | 3 | 3 | 0.5 | 3 | 3 | 0.5 | 0.2 | 0.2 | 0.2 | 0.3 |

| Secondary crosslinking | | | | | | | | | | | | |
|---|---|---|---|---|---|---|---|---|---|---|---|---|
| | Temperature [°C] | | 240 | 240 | 240 | 240 | 240 | 240 | - | - | - | * |
| | Time [h] | | 3 | 3 | 3 | 3 | 3 | 3 | - | - | - | * |

| Heat resistance evaluation | | | | | | | | | | | | |
|---|---|---|---|---|---|---|---|---|---|---|---|---|
| | TMA | | A | A | A | A | A | A | C | C | B | C |
| | Hanging method | | | | | | | | | | | |
| | | 300°C 48h | A | A | A | A | A | A | A | A | A | A |
| | | 300°C 72h | A | A | A | A | A | A | C | A | A | A |
| | | 325°C 24h | A | A | A | A | A | A | - | A | A | A |
| | | 325°C 48h | A | A | A | A | A | A | - | C | A | A |
| | | 325°C 72h | A | A | A | A | A | A | - | - | C | A |

| | | | | | | | | | | | | |
|---|---|---|---|---|---|---|---|---|---|---|---|---|
| Note) *90°C 2 h → raise the temperature: 2 h → 200°C 4 h → raise the temperature: 2 h → 305°C 12 h | | | | | | | | | | | | |

The evaluation results shown in Tables 1 have demonstrated that the cross-linked fluorine-containing copolymer products (Examples 1 to 6) obtained using the fluorine-containing compound of the present invention have both excellent short-term and long-term heat resistance.

## Claims

1. A fluorine-containing compound represented by the following formula (1): 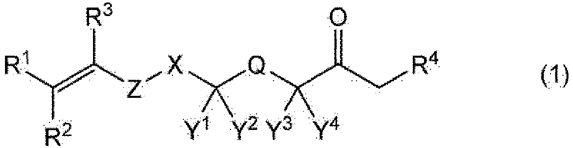 wherein
R¹ to R³ are each independently a hydrogen atom or a fluorine atom;
R⁴ is a hydrogen atom, a fluorine atom, or a C₁₋₈ monovalent organic group free from a carbonyl group;
Z is an oxygen atom, a sulfur atom, or -N(R⁵)- where R⁵ is a hydrogen atom or a C₁₋₄ monovalent organic group;
X is a single bond or a difluoromethylene group;
Y¹ to Y⁴ are each independently a fluorine atom or a trifluoromethyl group; and
Q is a C₁₋₈ perfluoroalkylene group optionally containing an etheric oxygen atom.

2. The fluorine-containing compound according to claim 1, wherein Z is an oxygen atom.

3. The fluorine-containing compound according to claim 1 or 2, wherein R¹ to R³ are each a fluorine atom and R⁴ is a hydrogen atom.

4. A fluorine-containing copolymer comprising: a constituent unit (A) derived from tetrafluoroethylene; a constituent unit (B) derived from perfluoro (alkyl vinyl ether); and a constituent unit (C) derived from the fluorine-containing compound according to claim 1.

5. The fluorine-containing copolymer according to claim 4, wherein the constituent unit (A) is from 29.9 to 80 mass%, the constituent unit (B) is from 19.9 to 70 mass%, and the constituent unit (C) is from 0.1 to 20 mass% based on total 100 mass% of the constituent units (A) to (C).

6. A fluorine-containing copolymer composition comprising the fluorine-containing copolymer according to claim 4 or 5 and a compound (D) having two or more structures (d) represented by the following formula (2): 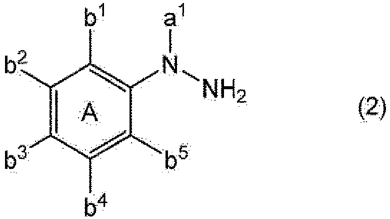 wherein
-a¹ represents a single bond and is attached to a hydrogen atom, a methyl group, or an optionally substituted aromatic ring; and
-b¹ to -b⁵ each represent a single bond, and at least one of -b¹ and -b⁵ is bonded to a hydrogen atom,
provided that the benzene ring in formula (2) is denoted as a benzene ring A.

7. The fluorine-containing copolymer composition according to claim 6, wherein the compound (D) has one or more structures in which the benzene ring A of one of the structures (d) is linked, via a single bond, to the benzene ring A of another structure (d).

8. The fluorine-containing copolymer composition according to claim 6, wherein the compound (D) has a content of from 0.1 to 10 parts by mass based on 100 parts by mass of the fluorine-containing copolymer.

9. A cross-linked fluorine-containing copolymer product obtainable from the fluorine-containing copolymer composition according to claim 6.
